# EUROPÄISCHE PATENTANMELDUNG

(11) **EP 0 998 932 A1**
(43) Veröffentlichungstag der Anmeldung: **10.05.2000**
(21) Anmeldenummer: 98119102.6
(22) Anmeldetag: 09.10.1998
(51) Int. Cl.: A61K 31/66, A61K 9/20, A61K 9/48

(54) **Feste pharmazeutische Darreichungsform enthaltend Diphosphonsäure oder deren Salze und Verfahren zu ihrer Herstellung**

(71) Anmelder: Roche Diagnostics GmbH, 68305 Mannheim (DE)
(72) Erfinder: Gabel, Rolf-Dieter, Dr., 68723 Schwetzingen (DE); Preis, Walter, Dipl.-Che.Ing., 67433 Neustadt (DE); Woog, Heinrich, Dr., 69514 Laudenbach (DE)

(57) **Zusammenfassung**

Die Erfindung betrifft eine feste pharmazeutische Darreichungsform, enthaltend als Wirkstoff eine Diphosphonsäure oder ein physiologisch verträgliches Salz davon, mit einer Auflösungsrate der pharmazeutischen Darreichungsform von mindestens 85% nach 30 Minuten, wobei der Wirkstoff als Granulat, gegebenenfalls zusammen mit pharmazeutischen Hilfsstoffen, in der inneren Phase vorliegt, und die äußere Phase als Gleitmittel weniger als 5 Gew.-% Stearinsäure, bezogen auf das Gesamtgewicht der Darreichungsform, enthält.

## Beschreibung

Die Erfindung betrifft eine feste pharmazeutische Darreichungsform, die als Wirkstoff eine Diphosphonsäure oder ein physiologisch verträgliches Salz davon enthält und in der äußeren Phase Stearinsäure als Gleitmittel beinhaltet. Die Erfindung betrifft weiterhin ein Verfahren zur Herstellung dieser Darreichungsform, deren Auflösungsrate nach 30 Minuten mindestens 85% beträgt sowie die Verwendung von Stearinsäure.

Pharmazeutische Darreichungsformen von Diphosphonsäuren sind zur Behandlung von Kalziumstoffwechselerkrankungen bekannt. Arzneimittel mit diesen Wirkstoffen werden zur Behandlung der Hyperkalzämie verwendet, aber auch zur Behandlung von Tumorosteolyse infolge von knochenmetastasen. Darüber hinaus können sie ebenfalls erfolgreich zur Behandlung von Osteoporose und Osteoporoseschmerz eingesetzt werden.

Da zur Behandlung dieser Art von Erkrankungen die Wirkstoffe häufig über einen längeren Zeitraum gegeben werden müssen, ist vor allem eine orale Applikation vorteilhaft, da sie in der Regel auf eine höhere Akzeptanz beim behandelten Patienten trifft.

Oral applizierbare Darreichungsformen sind für einige Diphosphonsäuren und deren Salze bekannt. So sind in EP-B 0 275 468, EP-B 0 625 355 (beide Boehringer Mannheim) und WO 93/21907 (Leiras Oy) pharmazeutische Zubereitungen mit Clodronsäure (Dichlormethylendiphosphonsäure) bzw. ihren Salzen beschrieben. Aus WO 93/09785 (Procter & Gamble Pharmaceuticals) sind orale Darreichungsformen für Risedronat (Salz der 3-Pyridyl-1-hydroxyethyliden-1,1-diphosphonsäure) bekannt. Dabei beschreiben WO 93/21907 und WO 93/09785 orale Darreichungsformen, die mit einem Überzug versehen sind, der sich erst bei pH-Werten über 5 bzw. 5,5 löst. Damit soll gewährleistet werden, daß die Darreichungsformen den Magen passieren und erst im Intestinaltrakt eine Freisetzung des Wirkstoffes erfolgt.

Die im Stand der Technik beschriebenen, festen Darreichungsformen von Diphosphonsäuren oder deren Salzen beinhalten ausgewählte pharmazeutische Hilfsstoffe in der inneren Phase, mit denen der Wirkstoff kompatibel sein muß, und ausgewählte pharmazeutische Hilfsstoffe in der äußeren Phase, die insbesondere eine gute Verarbeitbarkeit der Zubereitung auf einer Kapselfüllmaschine oder Tablettenpresse gewährleisten sollen. So beschreibt EP-B 0 275 468 Clodronat-haltige Arzneimittel mit einem hohen Wirkstoffgehalt von 80-95%, einem Füllstoffanteil von 2-10% und einem Gleitmittelanteil von 0-5% im Granulat, dem als äußere Phase zusätzlich ein Gleitmittel in einer Menge von 1-5% beigemischt ist, vorzugsweise Magnesiumstearat und Talkum.

Im Rahmen der Entwicklung sowohl einer Kapsel als auch einer Tablette oder einer anderen festen Arzneiform wird in aller Regel den Hilfsstoffen in der äußeren Phase ganz besondere Aufmerksamkeit geschenkt.

Von ganz besonderer Bedeutung ist hierbei die Wahl und Menge eines geeigneten Schmier- oder Gleitmittels in der äußeren Phase, welches großen Einfluß auf die physikalischen Eigenschaften der zu entwickelnden Darreichungsformen hat. Von der richtigen Auswahl und Menge hängt es ab, ob sich die Kapselfüllmasse oder die Tablettenmasse über einen längeren Zeitraum problemlos auf einer entsprechenden Maschine verarbeiten läßt oder ob es zum Kleben der Tabletten an den Preßwerkzeugen der Maschine kommt. Deshalb muß der äußeren Phase ausreichend Gleitmittel zugesetzt werden. Ist die Menge an Gleitmittel jedoch zu groß, können andere negative Effekte eintreten. So kann beispielsweise das Granulat soweit hydrophobiert werden, daß die daraus resultierende Arzneiform nur langsam zerfällt und die gewünschte Auflösungsrate (nahezu vollständige Freisetztung des Wirkstoffes nach 30 Minuten) nicht erreicht wird.

Bekannte Gleitmittel, die in der äußeren Phase Anwendung finden können, sind Magnesiumstearat, Calciumstearat, Talkum, Natriumstearylfumarat, Macrogol, hydrierte Ester von Fettsäuren mit Glycerin und Stearinsäure.

So werden in EP-B 0 275 468, das orale Darreichungsformen für Clodronat beschreibt, Magnesiumstearat und Talkum zusammen als Gleitmittel in der äußeren Phase eingesetzt, EP-B 0 625 355 (Boehringer Mannheim GmbH) offenbart für Clodronat-Darreichungsformen Magnesiumstearat als alleiniges Gleitmittel in der äußeren Phase. WO 93/21907 (Leiras Oy, Clodronat) beschreibt in Beispiel 1 die Verwendung von Talkum und Magnesiumstearat als Gleitmittel in der äußeren Phase und Stearinsäure als Gleitmittel in der inneren Phase. WO 93/09785 (Procter & Gamble, Risedronat) offenbart in Beispiel 3 Stearinsäure als Gleitmittel in einer Menge von 5,8 Gew.-%, bezogen auf den Tablettenkern.

Es zeigte sich jedoch, daß insbesondere bei geringen Wirkstoffmengen die eingesetzten Gleitmittel beziehungsweise deren Konzentrationen nicht optimal sind, da Auflösungsraten von 85% nach 30 Minuten, die eine gleichmäßige und nahezu vollständige Freisetzung des Wirkstoffes bedeuten, nicht erreicht werden bzw. die Auflösungsraten nach Temperaturbelastung, die über der der Raumtemperatur liegt, rapide absinken.

Der Erfindung lag deshalb die Aufgabe zugrunde, eine pharmazeutische Darreichungsform enthaltend als Wirkstoff Diphosphonsäuren oder deren physiologisch verträgliche Salze zu entwickeln, die so stabil ist, daß der Wirkstoff gleichmäßig und nahezu vollständig innerhalb von 30 Minuten freigesetzt wird und auch nach Temperaturbelastung keine Herabsetzung der Auflösungsrate eintritt. Dies soll sowohl für hohe als auch für geringe Wirkstoffanteile in der Darreichungsform gelten.

Überraschend wurde festgestellt, daß Darreichungsformen, die als Gleitmittel Stearinsäure zu weniger als 5 Gewichtsprozent in der äußeren Phase enthalten, Auflösungsraten von mindestens 85% nach 30 Minuten zeigen, und diese sich selbst nach mehrwöchiger Temperaturbelastung von 40-50°C nicht verändern. Dies gilt sowohl für niedrige als auch für höhere Wirkstoffanteile in der Darreichungsform.

Mit weniger als 5 Gew.-% Stearinsäure in der äußeren Phase, bezogen auf das Gesamtgewicht der Darreichungsform, wird einerseits eine ausreichende Gleitmittelwirkung erzielt, so daß die Tabletten- oder Kapselfüllmasse nicht an den Verarbeitungsmaschinen klebt, und andererseits wird eine Hydrophobierung des Wirkstoff-Granulates vermieden.

Bevorzugt ist Stearinsäure in einer Menge von 0,1 bis 3%, insbesondere von 0,9 bis 3%, bezogen auf das Gesamtgewicht der Darreichungsform, in der äußeren Phase enthalten. Besonders bevorzugt wird Stearinsäure in einer Menge von 1,5 bis 2,7%, bezogen auf das Gesamtgewicht der Darreichungsform, eingesetzt, wobei in diesem Fall die Freisetzungsrate mindestens 90% beträgt (bestimmt nach der Paddle-Methode nach USP).

Das Wirkstoff-Granulat kann pharmazeutisch akzeptable Hilfs- und/oder Zusatzstoffe, wie z. B. Lactose, Stärke, Glucose, Mannit, Calciumcarbonat, Calciumphosphat, mikrokristalline Cellulose, Hydroxypropylmethylcellulose oder andere für diesen Zweck in der Technik bekannte Substanzen enthalten.

Die erfindungsgemäße Darreichungsform kann in der äußeren Phase ebenfalls weitere pharmazeutische Hilfsstoffe enthalten, insbesondere ein Sprengmittel, wobei alle bekannten Sprengmittel zur Anwendung kommen können. Insbesondere hat sich als Sprengmittel für die Zwecke der Erfindung Polividon, quervernetzt, bewährt.

Bevorzugte erfindungsgemäße Wirkstoffe sind Ibandronat, Etidronat, Clodronat, Risedronat, Pamidronat oder Alendronat bzw. deren freie Säuren. Der Wirkstoffanteil in der erfindungsgemäßen Darreichungsform kann bis zu 95 Gew.%, bezogen auf das Gesamtgewicht der Darreichungsform, betragen. Besonders bevorzugt sind Wirkstoffgehalte von 1-30 Gew.%, bezogen auf das Gesamtgewicht der Darreichungsform. Mit der erfindungsgemäßen Lösung lassen sich ganz besonders bevorzugt orale Darreichungsformen mit Wirkstoffmengen von 0,5-100mg pro Darreichungsform, insbesondere≤ 50 mg pro Dosis, realisieren.

Ganz besonders bevorzugt ist die erfindungsgemäße Rezeptur für eine Darreichungsform mit dem Wirkstoff Ibandronsäure (1-Hydroxy-3-(N-methyl-N-pentyl)aminopropyl-1,1-diphosphonsäure) bzw. mit deren physiologisch verträglichen Salzen.

Zur Herstellung der erfindungsgemäßen Darreichungsform werden die Komponenten trocken gemischt. Der Wirkstoff, gegebenenfalls unter Zusatz von pharmazeutisch akzeptablen Hilfs- und Zusatzstoffen, wird mit einem üblichen Bindemittel, wie Stärkekleister, Polividon K25 oder auch nur Wasser feucht granuliert. Danach wird das feuchte Granulat getrocknet und gesiebt.

Anschließend wird die äußere Phase zugemischt. Entweder werden die Komponenten der äußeren Phase (Stearinsäure und Hilfsstoffe) zunächst miteinander gemischt und in einem weiteren Mischschritt zum Granulat zugemischt oder die Stearinsäure und gegebenenfalls weitere Hilfsstoffe der äußeren Phase werden einzeln direkt dem Granulat zugemischt.

Die erfindungsgemäße Mischung läßt sich leicht auf automatischen Anlagen abfüllen und wird anschließend zu Tabletten verpreßt oder in übliche Gelatinekapseln abgefüllt.

Tabletten beziehungsweise Kapselgrößen werden vorzugsweise so gewählt, daß eine Wirkstoffkonzentration von 0,5 - 100 mg pro Dosis erhalten wird. Je nach biologischer Potenz der Wirkstoffe und evtl. Hilfstoffen, die diese erhöhen können, bestimmt sich so die Größe der erfindungsgemäßen Darreichungsformen.

Die erfindungsgemäß hergestellten Darreichungsformen mit weniger als 5 Gew.-% Stearinsäure in der äußeren Phase ergeben gut rieselfähige und abfüllbare Kompositionen und haften nicht an den Werkzeugen beim Verpressen oder Abfüllen in Kapseln. Dadurch wird eine einwandfreie Dosierung der Einzeldosis mit einer Abweichung von weniger als 2%, in der Regel weniger als 1%, vom Sollgewicht erreicht.

In vergleichenden Untersuchungen mit dem Gleitmittel Magnesiumstearat in identischen Mengen in der äußeren Phase wurde eine in vitro Freisetzungsrate von 56% nach 30 Minuten bestimmt. Wurden diese Kapseln außerdem einer Temperaturbelastung von 40-50°C im Trockenschrank über mehrere Wochen ausgesetzt und danach erneut die Freisetzungsrate bestimmt, so ging der 30 Minuten-Wert auf unter 30% zurück.

Gegenstand der Erfindung ist somit auch die Verwendung von weniger als 5 Gew.-% Stearinsäure als Gleitmittel in der äußeren Phase einer stabilen, festen pharmazeutischen Darreichungsform, die eine Diphosphonsäure oder ein physiologisch verträgliches Salz davon als Wirkstoff enthält und deren Auflösungsrate nach 30 Minuten mindestens 85% beträgt, vorzugsweise mindestens 90%.

Die erfindungsgemäßen stabilen pharmazeutischen Darreichungsformen werden in den nachfolgenden Beispielen beschrieben, ohne daß durch sie die Erfindung beschränkt werden soll.

### Ausführungsbeispiele

### Vergleichsbeispiel 1:

Herstellung von Kapseln der Dosierung 5,0 mg mit 1,8 Gew.-% Magnesiumstearat als Gleitmittel

| **Pos.** | **Zusammensetzung** | **(mg/Stück)** |
|---|---|---|
| 01 | Ibandronat, Na-Salz | 5,682 |
| 02 | Lactose 200 (D 80) | 19,318 |
| 03 | Lactose D 30 | 249,000 |
| 04 | Polividon K 25 | 9,000 |
| 05 | Lactose D 30 | 128,000 |
| 06 | Polividon, quervernetzt | 25,000 |
| 07 | Magnesiumstearat | 8,000 |
| | **Gewicht** | **444,000** |

Die Menge des Wirkstoffes ist äquivalent zu 5,0 mg freier Säure.

### Verarbeitung:

Vom Wirkstoff (Pos. 01) wird zusammen mit Lactose 200 (Pos.02) eine Vormischung hergestellt. Anschließend wird die Vormischung zusammen mit weiteren Hilfsstoffen, wie Lactose D 30 (Pos. 03) unter Verwendung des Binders Polividon (Pos. 04) feucht granuliert. Dem getrockneten und gesiebten Granulat wird anschließend weitere Lactose (Pos. 05) zugemischt. Danach werden die Zusatzstoffe der äußeren Phase (Pos. 06, 07) einzeln der Mischung zugemischt.

Die so hergestellte Masse wird auf geeigneten Maschinen in Kapseln abgefüllt. Die im Rahmen der Inprozeßkontrolle untersuchten Kapseln weisen unmittelbar nach der Herstellung eine in vitro Freisetzung von 56% nach 30 Minuten auf.

Die Freisetzungsrate wurde nach der Paddle-Methode nach USP bestimmt.

### Vergleichsbeispiel 2:

Herstellung von Kapseln der Dosierung 5,0 mg mit 0,91 Gew.-% Magnesiumstearat als Gleitmittel

| **Pos.** | **Zusammensetzung** | **(mg/Stück)** |
|---|---|---|
| 01 | Ibandronat, Na-Salz | 5,682 |
| 02 | Lactose D 80 | 19,318 |
| 03 | Lactose D 30 | 249,000 |
| 04 | Polividon K25 | 9,000 |
| 05 | Lactose D 30 | 128,000 |
| 06 | Polividon, quervernetzt | 25,000 |
| 07 | Magnesiumstearat | 4,000 |
| | **Gewicht** | **440,000** |

Die Herstellung der Kapseln erfolgt analog Vergleichsbeispiel 1.

Das Ergebnis der in vitro Freisetzung nach 30 Minuten beträgt 56%.

Die Kapseln nach Vergleichsbeispiel 1 und 2 wurden einer Temperaturbelastung von 50°C im Trockenschrank über einen Zeitraum von mehreren Wochen ausgesetzt und danach erneut die Auflösungsrate bestimmt. Diese ging auf einen 30 Minuten-Wert von unter 30% zurück.

### Beispiel 1:

Herstellung von erfindungsgemäßen Kapseln der Dosierung 5,0 mg mit 0,9 und 1,8 Gew.-% Stearinsäure als Gleitmittel

| **Pos.** | **Zusammensetzung** | **a) (mg/Stück)** | **b) (mg/Stück)** |
|---|---|---|---|
| 01 | Ibandronat,Na-Salz | 5,682 | 5,682 |
| 02 | Lactose D 80 | 19,318 | 19,318 |
| 03 | Lactose D 30 | 249,000 | 249,000 |
| 04 | Polividon K25 | 9,000 | 9,000 |
| 05 | Lactose D 30 | 128,000 | 128,000 |
| 06 | Polividon, quervernetzt | 25,000 | 25,000 |
| 07 | Stearinsäure | (0,9%)4,000 | (1,8%)8,000 |
| | **Gewicht** | **440,000** | **444,000** |

Die Herstellung der Kapselfüllmasse erfolgt analog den Vergleichsbeispielen 1 und 2. Wie in diesen Beispielen bilden die Zusatzstoffe 06 und 07 die äußere Phase.

Die getrocknete und gesiebte Masse wird anschließend in Kapseln der Größe 0 abgefüllt.

Das Ergebnis der in vitro Freisetzung nach 30 Minuten beträgt
a) bei dem Ansatz mit 4,0 mg Stearinsäure: 90% und
b) bei dem Ansatz mit 8,0 mg Stearinsäure: 101%

Die Kapseln nach dem vorliegenden Beispiel 1 wurden ebenfalls einer Temperaturbelastung von 50°C im Trockenschrank über einen Zeitraum von mehreren Wochen ausgesetzt. Die anschließend bestimmten Auflösungsraten zeigten die gleichen Werte wie vor der Temperaturbelastung.

### Beispiel 2:

Herstellung von erfindungsgemäßen Tabletten der Dosierung 20 mg mit 2,5 Gew.-% Stearinsäure als Gleitmittel

| **Pos.** | **Zusammensetzung** | **(mg/Stück)** |
|---|---|---|
| 01 | Ibandronat, Na-Salz | 21,380 |
| 02 | Lactose D 30 | 45,520 |
| 03 | Hydroxypropylmethylcellulose | 2,000 |
| 04 | Cellulose, mikrokrisallin | 3000 |
| 05 | Polividon, quervernetzt | 5,500 |
| 06 | Stearinsäure | (2,5%)2,000 |
| | **Gewicht** | **79,400** |

### Verarbeitung:

Der Wirkstoff wird zusammen mit den Hilfsstoffen (Pos. 02,03,04) gemischt und mit Wasser feucht granuliert. Dem getrockneten und gesiebten Granulat wird eine Mischung der äußeren Phase (Pos. 05,06) zugemischt. Die preßfertige Masse wird anschließend zu Tabletten verpreßt.

Die auf diese Weise hergestellten Tabletten werden unmittelbar nach der Herstellung auf die in vitro Freisetzungsrate überprüft. Der Wert nach 30 Minuten beträgt 102%.

### Beispiel 3:

Herstellung von erfindungsgemäßen Tabletten der Dosierung 50 mg mit 2,5 Gew.-% Stearinsäure als Gleitmittel

| **Pos.** | **Zusammensetzung** | **(mg/Stück)** |
|---|---|---|
| 01 | Ibandronat, Na-Salz | 53,450 |
| 02 | Lactose D 30 | 113,800 |
| 03 | Hydroxypropylmethylcellulose | 5,000 |
| 04 | Cellulose, mikrokristallin | 7,500 |
| 05 | Polividon, quervernetzt | 13,750 |
| 06 | Stearinsäure | (2,5%)5,000 |
| | **Gewicht** | **198,500** |

### Verarbeitung:

Der Wirkstoff wird zusammen mit den Hilfsstoffen (Pos. 02,03,04) gemischt und mit Wasser feucht granuliert. Dem getrockneten und gesiebten Granulat werden die Bestandteile der äußeren Phase (Pos. 05,06) einzeln zugemischt. Die preßfertige Masse wird anschließend zu Tabletten verpreßt.

Im Rahmen einer Stabilitätsprüfung bei verschiedenen Temperaturen bis 40°C wurde die Bestimmung der Freisetzungsrate in unterschiedlichen Zeitabständen wiederholt. Selbst bei Temperaturen von 40°C waren nach 26 Wochen keine Unterschiede zur anfänglichen Freisetzungsrate festzustellen.

## Patentansprüche

1. Feste pharmazeutische Darreichungsform, enthaltend als Wirkstoff eine Diphosphonsäure oder ein physiologisch verträgliches Salz davon, mit einer Auflösungsrate der pharmazeutischen Darreichungsform von mindestens 85% nach 30 Minuten, wobei der Wirkstoff als Granulat, gegebenenfalls zusammen mit pharmazeutischen Hilfsstoffen, in der inneren Phase vorliegt, und die äußere Phase als Gleitmittel weniger als 5 Gew.-% Stearinsäure, bezogen auf das Gesamtgewicht der Darreichungsform, enthält.

2. Darreichungsform nach Anspruch 1,
dadurch gekennzeichnet,
daß sie in der äußeren Phase 0,1 bis 3 Gew.-% Stearinsäure, bezogen auf das Gesamtgewicht der Darreichungsform, enthält, vorzugsweise 0,9 bis 3 Gew.-%.

3. Darreichungsform nach Anspruch 1 oder 2,
dadurch gekennzeichnet,
daß sie Stearinsäure in einer Menge von 1,5 bis 2,7 Gew.-%, bezogen auf das Gesamtgewicht der Darreichungsform, enthält und die Freisetzungsrate nach 30 Minuten mindestens 90% beträgt.

4. Darreichungsform nach einem der Ansprüche 1 bis 3,
dadurch gekennzeichnet,
daß sie als Wirkstoff Ibandronat, Etidronat, Clodronat, Risedronat, Pamidronat oder Alendonat enthält.

5. Darreichungsform nach einem der Ansprüche 1 bis 4,
dadurch gekennzeichnet,
daß der Wirkstoff in einer Menge von 0,5-100mg pro Darreichungsform, vorzugsweise≤50mg pro Darreichungsform, enthalten ist.

6. Darreichungsform nach einem der Ansprüche 1 bis 5,
dadurch gekennzeichnet,
daß in der äußeren Phase als weiterer pharmazeutischer Hilfsstoff ein Sprengmittel enthalten ist.

7. Darreichungsform nach Anspruch 6,
dadurch gekennzeichnet,
daß das Sprengmittel Polividon, quervernetzt, ist.

8. Verfahren zur Herstellung einer festen pharmazeutischen Darreichungsform nach den Ansprüchen 1 bis 7,
dadurch gekennzeichnet,
daß man den Wirkstoff mit pharmazeutischen Hilfsstoffen nach an sich bekannten Methoden zu einem Granulat verarbeitet, dieser inneren Phase weniger als 5 Gew.-% Stearinsäure als Gleitmittel und gegebenenfalls weitere Hilfsstoffe zumischt und diese Mischung in Kapseln füllt oder zu Tabletten verpreßt.

9. Verfahren nach Anspruch 8,
dadurch gekennzeichnet,
daß man die Stearinsäure und gegebenenfalls weitere Hilfsstoffe der äußeren Phase einzeln dem Granulat zumischt.

10. Verfahren nach Anspruch 8,
dadurch gekennzeichnet,
daß man zuerst die Stearinsäure mit weiteren Hilfsstoffen der äußeren Phase vermischt und dann diese Mischung dem Granulat zumischt.

11. Verwendung von weniger als 5 Gew.-% Stearinsäure als Gleitmittel in der äußeren Phase einer festen pharmazeutischen Darreichungsform, die eine Diphosphonsäure oder ein physiologisch verträgliches Salz davon als Wirkstoff enthält und deren Auflösungsrate nach 30 Minuten mindestens 85% beträgt, vorzugsweise mindestens 90%.

12. Verwendung nach Anspruch 11,
dadurch gekennzeichnet,
daß 0,1 - 3 Gew.-% Stearinsäure, bezogen auf das Gesamtgewicht der Darreichungsform, eingesetzt werden.
